# EUROPEAN PATENT SPECIFICATION

(11) **EP 2 821 487 B1**
(45) Date of publication and mention of the grant of the patent: **26.10.2016**
(21) Application number: 13755864.9
(22) Date of filing: 26.02.2013
(51) Int. Cl.: C12N 15/09, C12Q 1/68, G01N 27/62

(54) **METHOD FOR DETERMINING PRESENCE OR ABSENCE OF CANCER CELL DERIVED FROM HEPATOCELLULAR CARCINOMA, AND DETERMINATION MARKER AND KIT**
VERFAHREN ZUR BESTIMMUNG DES VORHANDENSEINS ODER NICHTVORHANDENSEINS VON KREBSZELLEN AUS EINEM HEPATOZELLULÄREN KARZINOM SOWIE BESTIMMUNGSMARKER UND KIT
PROCÉDÉ POUR DÉTERMINER LA PRÉSENCE OU L'ABSENCE DE CELLULE CANCÉREUSE ISSUE D'UN CARCINOME HÉPATOCELLULAIRE, ET MARQUEUR DE DÉTERMINATION ET TROUSSE

(30) Priority: 29.02.2012 JP 2012044324; 19.09.2012 JP 2012205775
(43) Date of publication of application: 07.01.2015
(73) Proprietor: Sysmex Corporation, Kobe-shi, Hyogo 651-0073 (JP); The University of Tokyo, Bunkyo-ku, Tokyo 113-8654 (JP)
(72) Inventor: SAKAI, Ayako, Hyogo 6510073 (JP); NAGAE, Genta, Tokyo 113-8654 (JP); MIDORIKAWA, Yutaka, Tokyo 113-8654 (JP); KAJITA, Masahiro, Gunma 3700033 (JP); ABURATANI, Hiroyuki, Tokyo 113-8654 (JP)
(74) Representative: Paemen, Liesbet R.J.
(86) International application number: PCT/JP2013/054961
(87) International publication number: WO 2013/129397

(56) References cited:
- WO-A2-01/77376
- WO-A2-2008/100913
- JP-A- 2011 177 139
- STEFANSKA BARBARA ET AL: "Definition of the Landscape of Promoter DNA Hypomethylation in Liver Cancer", CANCER RESEARCH, vol. 71, no. 17, September 2011 (2011-09), pages 5891-5903, XP002747487, ISSN: 0008-5472
- LIU HONGYAN ET AL: "DNA Methylation Suppresses Expression of the Urea Cycle Enzyme Carbamoyl Phosphate Synthetase 1 (CPS1) in Human Hepatocellular Carcinoma", AMERICAN JOURNAL OF PATHOLOGY, vol. 178, no. 2, February 2011 (2011-02), pages 652-661, XP002747488,
- DATABASE BIOSIS [Online] BIOSCIENCES INFORMATION SERVICE, PHILADELPHIA, PA, US; November 2007 (2007-11), KUANG SHAO-QING ET AL: "Eph receptor tyrosine kinases and ephrin ligands are epigenetically inactivated in acute lymphoblastic leukemia and are potential new tumor suppressor genes in human leukemia", XP008177843, Database accession no. PREV200800217400 & BLOOD, vol. 110, no. 11, Part 1, November 2007 (2007-11), page 634A, 49TH ANNUAL MEETING OF THE AMERICAN-SOCIETY-OF-HEMATOLOGY; ATLANTA, GA, USA; DECEMBER 08 -11, 2007 ISSN: 0006-4971
- LIU S ET AL: "Use of protein array technology to investigate receptor tyrosine kinases activated in hepatocellular carcinoma", EXPERIMENTAL AND THERAPEUTIC MEDICINE, vol. 2, no. 3, May 2011 (2011-05), pages 399-403, XP002747489, SPANDIDOS PUBLICATIONS LTD. GRC ISSN: 1792-0981
- SUN Q ET AL: "Expression profiling reveals dysregulation of cellular cytoskeletal genes in HBx-induced hepatocarcinogenesis", CANCER BIOLOGY AND THERAPY, vol. 6, no. 5, May 2007 (2007-05), pages 668-674, XP055221793, US ISSN: 1538-4047
- ISSA JEAN-PIERRE: "CpG island methylator phenotype in cancer", NATURE REVIEWS, vol. 4, no. 12, 1 December 2004 (2004-12-01), pages 988-993, XP002410376, CANCER, NATUR PUBLISHING GROUP, LONDON, GB ISSN: 1474-175X, DOI: 10.1038/NRC1507
- DATABASE BIOSIS [Online] BIOSCIENCES INFORMATION SERVICE, PHILADELPHIA, PA, US; March 2011 (2011-03), JUNG H Y ET AL: "OVEREXPRESSION OF THE RECEPTOR TYROSINE KINASE EPHA4 IN ADJACENT NONNEOPLASTIC LIVER IS ASSOCIATED WITH POOR PROGNOSIS OF HEPATOCELLULAR CARCINOMA PATIENTS", XP008177854, Database accession no. PREV201200047386 & JOURNAL OF HEPATOLOGY, vol. 54, no. Suppl. 1, March 2011 (2011-03), pages S389-S390, 46TH ANNUAL MEETING OF THE EUROPEAN-ASSOCIATION-FOR-THE-STUDY-OF-THE- LIVER (EASL); BERLIN, GERMANY; MARCH 30 -APRIL 03, 2011 ISSN: 0168-8278
- DENG, Y.B. ET AL.: 'Identification of genes preferentially methylated in hepatitis C virus-related hepatocellular carcinoma.' CANCER SCI. vol. 101, no. 6, 2010, pages 1501 - 1510, XP055163819
- TAO, R. ET AL.: 'Methylation profile of single hepatocytes derived from hepatitis B virus- related hepatocellular carcinoma.' PLOS ONE vol. 6, no. 5, 2011, page E19862, XP055163821
- SHIN, S.H. ET AL.: 'Identification of novel methylation markers in hepatocellular carcinoma using a methylation array.' J. KOREAN MED. SCI. vol. 25, no. 8, 2010, pages 1152 - 1159, XP055163822
- HERNANDEZ-VARGAS, H. ET AL.: 'Hepatocellular carcinoma displays distinct DNA methylation signatures with potential as clinical predictors.' PLOS ONE vol. 5, no. 3, 2010, page E9749, XP055163824
- BIBIKOVA, M. ET AL.: 'High-throughput DNA methylation profiling using universal bead arrays.' GENOME RES. vol. 16, no. 3, 2006, pages 383 - 393, XP002445638

### SEQUENCE LISTING

<110> SYSMEX CORPORATION THE UNIVERSITY OF TOKYO
<120> Method for detecting presence or absence of cancer cells derived from hepatocellular carcinoma, and determination marker and kit
<130> SYSM-089-EP
<150> JP 2012-44324
   <151> 2012-02-29
<150> JP 2012-205775
   <151> 2012-09-19
<160> 35
<170> PatentIn version 3.5
<210> 1
   <211> 22
   <212> DNA
   <213> Artificial Sequence
<220>
   <223> primer
<400> 1
   gggtttagtt ataggtttgt tt 22
<210> 2
   <211> 22
   <212> DNA
   <213> Artificial Sequence
<220>
   <223> primer
<400> 2
   accctaactc rctattatca ct 22
<210> 3
   <211> 23
   <212> DNA
   <213> Artificial Sequence
<220>
   <223> primer
<400> 3
   gtatattttg gaatttgtga tag 23
<210> 4
   <211> 23
   <212> DNA
   <213> Artificial Sequence
<220>
   <223> primer
<400> 4
   ctctcctaac ttaaacattt att 23
<210> 5
   <211> 20
   <212> DNA
   <213> Artificial Sequence
<220>
   <223> primer
<400> 5
   gaggtttagg ttttgtggag 20
<210> 6
   <211> 20
   <212> DNA
   <213> Artificial Sequence
<220>
   <223> primer
<400> 6
   tacccaacac aatacaccaa 20
<210> 7
   <211> 10
   <212> DNA
   <213> Artificial Sequence
<220>
   <223> Tag sequence
<400> 7
   aggaagagag 10
<210> 8
   <211> 31
   <212> DNA
   <213> Artificial Sequence
<220>
   <223> T7-promoter sequence
<400> 8
   cagtaatacg actcactata gggagaaggc t 31
<210> 9
   <211> 23
   <212> DNA
   <213> Artificial Sequence
<220>
   <223> primer
<400> 9
   tttatttttg agtaggtaga tgt 23
<210> 10
   <211> 21
   <212> DNA
   <213> Artificial Sequence
<220>
   <223> primer
<400> 10
   aaacctaaaa caaatcctac c 21
<210> 11
   <211> 26
   <212> DNA
   <213> Artificial Sequence
<220>
   <223> primer
<400> 11
   ttatagagtt tagttgggtg tatagt 26
<210> 12
   <211> 20
   <212> DNA
   <213> Artificial Sequence
<220>
   <223> primer
<400> 12
   aacacactct caacccacct 20
<210> 13
   <211> 25
   <212> DNA
   <213> Artificial Sequence
<220>
   <223> primer
<400> 13
   gttttgtttt ttaaggggtg ttgag 25
<210> 14
   <211> 25
   <212> DNA
   <213> Artificial Sequence
<220>
   <223> primer
<400> 14
   acactaactc craaaactac aaaac 25
<210> 15
   <211> 21
   <212> DNA
   <213> Artificial Sequence
<220>
   <223> primer
<400> 15
   gttttatgtt gggagttttg a 21
<210> 16
   <211> 26
   <212> DNA
   <213> Artificial Sequence
<220>
   <223> primer
<400> 16
   atcctcttcc tactatctat ttactc 26
<210> 17
   <211> 4312
   <212> DNA
   <213> Homo sapiens
<400> 17
   gaggcttcag ggaggaaatg ccgggagagg aacagagcct ggaacagcga aagaaacact 60
   taaatgtcag aaatcaagcc gggcagaaaa tgactgggga aaggacggga ggagcacggg 120
   cgtcggccga gcctcacctg agtcatcttc tctctgttgg ccttggggtt caggggggcc 180
   tcggtcagca gcactgggtg ctcctccggg gccacgcgca gctcgttgta gaaggtgtgg 240
   tgccagatct tctccatgtc gtcccagttg gtgacgatgc catgctcaat ggggtacttc 300
   agggtcagga tgccacgctt gctctgggcc tcgtcgccca cgtaggagtc cttctggccc 360
   atgcccacca tgacgccctg caggggacga cccgtcagcc tcgccggcga caccgaaccc 420
   accccgcaac gcagaaccca ggagccccgc ggcgccatcc actcacctgg tgtctggggc 480
   gcccgacgat ggaaggaaac acggctcggg gagcgtcgtc cccagcaaaa ccagctttgc 540
   acatgccgga gccattgtca atgaccagcg cggcgatctc ttcttccatt gcgacctgcc 600
   cggaaaagga tggactcagg cgggcgcgtc tgtaacacgg tcccctcccc acagccacct 660
   aatgccctcc cgcggggaag cctcggccct gccccaaccc cagcggccgt ggcctccaag 720
   atcgcaaccg cctggaaccg aaggccgggc cttttacgta acgtccacgg ctcggaagtc 780
   tgcactgcgg ccgggccccg ccctggaccc ccggcgcccc cccagccccg tccgcctgac 840
   ccggcccacc ccgccttttg ttcccgggga aggcgcgacg aggcctcggc agctggaagc 900
   ggggccagcc ggggtcgggg ggcgcaggcc tgcgacgtcc agctcaggcc ccggggcggg 960
   gcgctcaccg gcagagaaac gcgacggcgg agcggcggaa gaacagagtg cgagagctgg 1020
   cagcggcgac tgagaccgac cgcggcctcc cccgccgtta tttaagcgga agcggcgcgc     1080
   ccgcgcggcc cggcggcgcg cgcggcccca gaacatgtcc atatatggcg atctttccga     1140
   aagccgggca cccattggcc cgccgggcag ggacacgtgg ggccccggcc cgccccgccc     1200
   ccagcccgca ccgcctccca acggccccgc ccgcgccacc cccgcgcgct cgcgaccctg     1260
   cgcgggccgg cgggcgggtc tgagttgggg cgccctccga gggtcgccgg gagggccgaa     1320
   gggctgacgg gcctggcccc tccccgggac tgccgcgccg tggggagggc cctgctgcgc     1380
   cccgaaactg cctgacccgg ggcggggccg cgccggagct ggggtgggtc cccgagtccc     1440
   cggccacgct gcggggcttt gctccctggg acgtcccttg caatctttcc cctcgggctc     1500
   cacgaggatc tcgggcgcca gtgccgggct gaggaagggg cgcggcgggg gtccgcgacg     1560
   ctctgcgtcc ctgggcgcgg gggagctctc aggagccctc acctcgcccc ggtcaccata     1620
   ggaaacagcc cgcccgtccc caagcgcagc caccaccatg caggtccctc cacggggcgc     1680
   cctgccccgg atctccgttc gtttgaagac cgaggcccct ttctacccga cccgacacat     1740
   ccctgcccct caaatcacag gcctgattgt ccccaggcgc gcgctccggg ttggggctac     1800
   cccgccccct tatgtgctga gaaggtggtg tggagtgagg cctttgtttc attctggtca     1860
   cctgtagaca ttatagggaa actgaggtag acaatgggag ccctgacttt tttgaaggtc     1920
   ggctccccag cttcgggcgg tcggggggat accctcttct gtttacgatg ctagttaaga     1980
   gcagcgatca gggctggcgt gggtttagcc acaggcttgt ccagggcgct cactcctcgc     2040
   caggagcggc gccggtgctt tggttacacc ccgagaggcg cccggcacta cggtttcctt     2100
   gtgcgctgct ggaattttca gggttggagc gccggccgcg taggggtggc agtgaggccg     2160
   cgcagtgagg ggacggactc gcccgaggtc gcagagcgtt cagattgact cgacaggctg     2220
   gggggcgggg ccggggtccc cacgcggagt ccggggccct gcaacgttag ctcgcgaccc     2280
   cgtgtttggg agtgacaaca gcgagtcagg gcaaggcggc cggcccagct cacctgcggg     2340
   ctgcagtcag cccggacgcc gagacccgtg cctaaggccc ccgtgcccca ggcctcccag     2400
   gggtgctgag cgcccccagg cccagaatct ccgggggccc gggcaaggct gtcaggtatg     2460
   ttccctcccg tggcgaagcc ggggctgggc ctccaacaga cccacccgga ctcgcgtccc     2520
   tgggcagaac cacacagcct gctcgacccc cgggtgagga cttccacttg gggggtccca     2580
   agatgcgacg tcacacaccg agggacacga ggcgggtggg gaatagccac aagcccctcc     2640
   atcctgcgtt caccccacac tacgttctgc tccgggccgg ccggggaccc tgcccctccg     2700
   ggtctctggt ctcctccgaa gcttctttgt ctaggaagtg cggggaggtg ccggccccgg     2760
   gcgcgcgtcc ccgtccccca cctttcttcc ccacgttgcg gacccgaggc cgccagggtc     2820
   gtgggggcgg gggcgaggct ggcgcctcga gacccccggg ttccccgcca cccgcagggc     2880
   ctgagccgcg gggaggggcg gggaggggac gccgccgcgg gccgggggtg gagagcgact     2940
   tcctcctccg cggccgcgtc acgcggcagg ggtcaaggtg tggcccaggt acgcggcggg 3000
   gggcggtggc gcggccatat ttggcgactt cggcgccgcc ggagccccgc ccgcctccgc 3060
   cgggaagccc ggcccggggc agcgccatcc ccgcccgccg ccgggcctga gtcacggcgg 3120
   gcggggggcg gaagtaacgg acggggggcg cccgcccggc gctcctggcc ttatttagtc 3180
   aaggaggccg ccgattggcg cgcgggacag aggggcggcg gccgggcagc ctcggggcgt 3240
   aggagtggcc cggcgcctcc gaggggtcgg ggcgggagcc gggcgtgggg ggcctcggag 3300
   cctggggagt cccgggaccg acggcggcgg ggatgcgggt ctgcctgtcc ctgggatgcg 3360
   ggcgggcggg cagggcgagg cctggagccc gcggggcgca gggggctgga ccgcgcgctg 3420
   tgatttttct gtttagcagc ggggcctcgc ccaaggtcgc gcggggcctg tcctggatat 3480
   gctctgggaa gccagtggcg cagggcagcc gccacccttc tggaaccagt gccggagagg 3540
   gccgggcagc tgggcctggg gaggggaccg catggatgga gctgggggta gctgggcggc 3600
   ctccggagcc gggagagtcg gcagctgcac ttccgccaga ggtgggtgtg tccttcacat 3660
   ttcaggaagg gagacttggg gcctggagaa gcgatgtgat ttttcttttc tagttcagtg 3720
   ctggttttga tggcttttta tcatgacctt gttatgtctt attttagttt cggcccattt 3780
   agtggatacg acaacagtgg cccagggagg tatggcagag ctgaggctta acccagggcc 3840
   tgcgccctcc acggcctgca ctgccccacc tccagctcct tgccctgttc ctccctctgc 3900
   accggatcag cccccggact ctgggtcacc tccacaccag ttgacagggc cccccagtcc 3960
   ccaccgccaa ccacctggcc ggctacttgt cagacagaca tgggggcgtg ggcatgggtc 4020
   cccaccccta gcctttgcct ctgtcactct acctgcctgg aatgtctact ttttctttat 4080
   attttatttt attgtatttt tgagacagtc tcattgtcgc ccaggctgga gcgcagtggc 4140
   gcgatcttgg ctcgctgcaa cctctgtctc cggggttcaa gcgattctcg ggccttagcc 4200
   tcccgagtag ctgagactac aggcgtgcac caccatgcct ggctaatttt tgcatttttg 4260
   gtggagacag ggtttcacca tgttggccag gctggcctga actcctgacc tt 4312
<210> 18
   <211> 4146
   <212> DNA
   <213> Homo sapiens
<400> 18
   aacgtgatgg gaatagaaag caacaaagtg aattaatttt taagggggag gtgggggagg      60
   ggaggggaac agacctccct ccctcccctt caacattagc ctagggctct cagagccttt     120
   ttgtcttggg ctcccaagtt tttctccccc ccccccaaaa aaagaggggg tttaaaaaaa     180
   agaaagaaaa cagtctctac atctggggcg agagaggtct caggggacgt cctaacaagt     240
   gtgtgaagtg gccaggagaa cgcgggatcc acgattcctc tcaggggaat gaataaccct     300
   agggaccgag gggcggcgac ggccgagatg gcaccagaac caccagaagc cacgtcggaa 360
   ggagatggga gatccagagg gcgagagagg gcccctttcc tcccaagcga tgtctgtggg 420
   caggtgtata aatctctcag agcctccgtc tggagggctc cgggtaccgg ctgcccagcc 480
   aaggctaagt tggtttttgg ctccctcctt cggaacagag aaaacgatag attacacagg 540
   gatggatgga cggatgtctg gagagatgga aagatgaatg gatggatgga tgggtggatg 600
   gatggataga tgaatgggtg gatggatgga gggatggatg gacggacgga cggacagata 660
   tatggatgga tgcattatgg atggatgcat ggacggacgg acggatgcac ggagagatgg 720
   atggatgcat ggatggatag atagacggat ggacggacgg atagatagat ggatgccgga 780
   aaggagaaga atgagagacg gatgtgagac tagatgcacg caggccagag caccagcata 840
   cgctggaaca gagcacgagc atacactcga acacacgcgc acacactcag gacatctgcg 900
   cacagacata caatcctccg cgtccgcttc cacgcaggca ttcgcgcacc tacatacacg 960
   cagttgcacg cgcgcgcaca cacacaggca cacacacgca gacatgcaca cacacgcaga 1020
   catgcacaca caccacacat gcagacatgc acacacacgc agacatgcac acacacacca 1080
   cacatacaca cacacacaca cacagttcgc ctctccctgg gtttctcaga aactaaatga 1140
   tcaccgcccc cccgccccgc cccacctccc gacacagaca cacaggcaca tacaatcctc 1200
   ccagcacgtc cgaacggatg cacagaaaac tgagcaccca gacgggtccc gtcgaccccg 1260
   cacgttagct ctaaactgtg tgcaattctg ggcgaaaaag ctaaaactcg actgcgttca 1320
   acttgccggc gggttcccca agtctgcggg gcgaagagct cgggcccctc aggcccgcaa 1380
   tcgcaccctc ggggcgctgg gcttggcgag gagaaaggtg tctgactccg ggtgctagaa 1440
   atcaggtcac tggcgcctga cgagcggcgc cacgaccgct gcgctgcgga gcaggccccg 1500
   cagcccggtc ccgagaagcg cagggctcgg gaaactttgc agaaaccaga gctcgaaagg 1560
   ctttcgctag aatccgggag caccaagcct tcactgtgct ccaggctgcg ttttcccctc 1620
   gccccgggct ggctcaggag aggacgtggt tcttgtaatt ttttttttaa atcccggcgt 1680
   tgtctcccgt gcatcccctc agggcgcctc gagcgggcct ctctggcgga tgctgataaa 1740
   ctttggcctt tggttacaaa cttggacaga cccgccgcgt gcacgggtca ccgcctcggg 1800
   gcaggctgtc cggcggttcc ccgctgcccc ttcgccgatc ccctcgcctc agcccccttc 1860
   tcccgaatcg ggacgcacca ttcacactgg gctccccccg caccacctgg ccctgcgctc 1920
   ctgaggaccc ctcacccgcg atggcccctc gctgtccccg accacagaaa ggccgtcccg 1980
   ctcttacctt cattcgcggg gtataccctg gaacctgtga cagcgtcgca aatcccgaag 2040
   agacacgaaa atagggcgaa atagaaaatc ccagccatgg ttcgccggtg ccaacgctgc 2100
   tcctgccgct tctatcccag tggaataaat gcttaagtta ggagagcagc gggctgaaga 2160
   cattgccaag ggggcgggcc cggccggtga cgtgagcccg ccagtccggg gcccgcggcc 2220
   aatggcggcg cagacagggc ggccgagccc cgccttcacc gagcagggcc cgccccaggg 2280
   ttccgccccc tccgggctcc acggggcgcg cggtctcccg gggctctagg gggcgagcac 2340
   ggccggtccc cgcccccgcc tgccggaggg agccagcggg atcccccacg ttacctcgaa 2400
   ggtcgagccc ctggacggcg gagggacttt ggaaaagctc ggaattgacc gaggggagcc 2460
   agagaagagg ttggaaacct tttctcccac cacgggcggt ttaagatgga ggaaaacgct 2520
   cttttattga aataaaagaa gtctaagctg acctgcggtt gcttctttag gagagtcgac 2580
   gtgtctgcgt ccagcgcgcc cgggctgagc tgcagactcg cgcctgccac cgccaccctc 2640
   tgccaccttg ccctgttttg tctttatggc gcttccccct ctgaaatact ctttgcgggg 2700
   agttttgtgg ctctatcact cgcttcttcc ctggctcccc agatgcctgg agagcgggga 2760
   cctacttggt ccccaacgtc cagtccgcag cagggctccg tcactgtctc cagagactga 2820
   acgaatgact ttctagcagt aaggaagtcc ccgctggccc cgtccccctg cacacgccgt 2880
   ggacctcgcc cagggcccag gacgccccga gatggccttg ggcagggaac cagttgtgga 2940
   tttcccgggg gcagggttgg gggcagtccc tggaaccggg cctagcccgt ctgggagcgg 3000
   tggggtagaa gtgaaaggct taagtgtggg tgcggggact tgcagccgca cagacaaggc 3060
   agaccgggac tgggaagctg cccggagcgg ctgctgcgac tctccgctcc gacctagccg 3120
   gggcagccct ccctgccccg ggagaagacg gcgagcagga ggacccgcag agccccgccc 3180
   ggctgctccg gccccggggg cactggcacc atcgggctgt ggtcttttcc cgatgcccga 3240
   gccgtggacg cggccaccta agagcggtcc ggcgaaggca gcgcagccgg tgtcccaggg 3300
   accggtcccc aacgccactc ggtgcatttc cccaggcgct ggcaggggca ctgattccta 3360
   gcaatgaggc ctcccttccc cttcccaggc cgcccctcct ctgactccct ccagagcagg 3420
   gccgagtttt cctggggagc ctgggtcccg gcggccgggc ggccgctggg aggtggagga 3480
   gcctctccag ccccggccgc agctcatcgt gagccaaccg cgccacctgc cggccagatg 3540
   cgggagtgtg cggccccgga ggaagggcga gcggacaaag accgcaaccc gcaatccgca 3600
   accccgcaga gaggtggtgc gtgcgtttgg gcgagctttt cagccaccgc caagtctcgt 3660
   gcactagggc tactcctacc gtggggctgc ggacagcgct caagaggtcc tggagtctgt 3720
   cgtgactctc gcctgctgga tttcaaaaga tggaatcgga aagcgtttca aggagaaact 3780
   cctaacaaac cttccggggg ttgcctgagt ggctgctctc ggaaaagcgg atcctaaata 3840
   aagcgggagg gttatagggc gacgtcgagg agaggacagg tctcgagtca ctgctacagt 3900
   ttcaggtcac tgggctccgc agcagatcgt gttttctccc gtggctcgag agctgcgctg 3960
   gtttctcatg caaactcaga gccgagctaa tgacatgagc aacttttact tttacacaag 4020
   atgagcacgc gtgccgaggc gctgggcggc ggctgtgtga gttggtggcc cagacgaaca 4080
   gcttgtgcga gactctgggc atttcggttt ctagatacaa gatttgctta aatgtcacag 4140
   tccaaa 4146
<210> 19
   <211> 4272
   <212> DNA
   <213> Homo sapiens
<400> 19
   ataactgaag gtggaacctg ggtagagggc tgctgcactg cagtaggtat gtttgcttgc      60
   tgaccaatat ttgcaatctg actgccagta ggtacagcag acactgctgc cggagcctgg     120
   ccaacaggct ggacagatgc ccctgcaggt tgtgctggga ctgctgtggg ctgcactggc     180
   agctggatac cctgtggctg agccacagga atcactgttg ttcctgctcc tactcctgca     240
   gaactgggct gtccggagga cattgctgtt tgaagaattg gctgctgttg tacatactct     300
   gaagcaggat tttgagtcac tgatttgaca tggcctgggg ccatctgtgt agaaaccatt     360
   ggttgctgtt gtccatactg taactgttgg ggtggtggtg cccctggaag gggagtttgc     420
   actggaggag ccgcctgaga atatggtagc tggggttgag ccaaactgga aatggaaggc     480
   tgctgaccta aagctgaagt tacaccaacc acatttacag gcgatggctg gataccagtt     540
   gcagcactca tagtggcttg cagaggtact ggctgaagac cttgcttttg ctgatagctc     600
   agttcttgag actgtaattg tacttgtgag atctgtgact gagaaatact ctgtggtata     660
   ctaactgctg gaatactctg tggaccagtg ctaccaaaat ccatctgttg gagggtcaca     720
   ccttggagag ctggttgttg ctgttgttgt tgttgttgct gctgctgctg ctgcaccacc     780
   acagtagggg ctcccatctc tccacttccc acactctctg tatagtgact cagtgtgctg     840
   acactactgc tcactgaact cccactagtg ctctcccttt cagaagtcac ttctatcgga     900
   ttttgcttta cagtctccac cactttattt atcagcacac cttctgtagc aggtacagca     960
   ttttcttttt catagaactc agtgcaagtc catctacctt ttttaaaggg ctcagaacta    1020
   gaatctaact tcacaactct gaacctcgaa gtgttaactg ttggttgttg ctgctgactt    1080
   gaaaccgatc ccccagtcat ccctgcagct gcattaggaa cactgctaac agcagcagag    1140
   gaagaaatag taccattgcc catgccactc aagatattca cattaacacc actggtaact    1200
   ccaggcccaa cactcacacc agcagcacta ggaatattgc ttgtacttat attaacatta    1260
   ccaagcatgc tgcttgtcac attaggatta aaactaccca cagcagtaat gttaacatta    1320
   tttactgtac tagtgccagt aacagaattt atacctattc cacctgtagt acttggagca    1380
   cgcatattag tcattacaga tgcaggtgaa ccactggatg atacagcaga agttggtgca    1440
   actggtgtga tactgtcaga gcttccagtt gtagagagtt ttctagatac tgggcttgag    1500
   ggtggcccac cagtaatgga tgcactggcc acagcaacat gagatggatg gtggtgacca    1560
   tgttggaggt ggtgcccatg atgaatctga tggtggtgat ggaggtggtg tggatgagca 1620
   ttcccattga tcacaacatt ctgttgtgga aggtgaggca aatgaggctg aggaaggtgg 1680
   ggctggttgg gagagactgc cccaggtgtc tcggcttcct ggaagttatt tagggtctct 1740
   tctgaggagc tgcgttcggg ctcccctaag tcagtagccc tggaaagtga cacatcaagg 1800
   atctccgaag aagagagatc ttccgtgtga gattcatcca gatcatcata gctctcagtg 1860
   tcctctgcta tactgttgtt agagctgata ctagcggaga tctgagcagg agtaacgcta 1920
   gttatctgga agccactttt ctttttcatt tgagttccgc ctggcgcaag aggctgtgcc 1980
   tgcagctgag cctgcgaaag gaggttcagg ctttgtggag gcggaggctg tggtcccgac 2040
   gtagaagatg ctgcaggggg cggcggctga agcagcgacg gaggcggaaa atcctcggaa 2100
   gatgtggcat tactaccgac gccggtacct gctgcattga gagcagaggc gctgccacta 2160
   ccgctgcccc ttcgagggaa cattgccggg tgcgccatct tcctagcgct aatgtctgca 2220
   gcggcggcgg ccgcggcggt ggactcaggc ggctggtgca ttgtgttggg taccgggggc 2280
   gcggaggaga cgagtgcaat ttccttctgc accgtaatct ttgtattgga gacgccggag 2340
   aggaaaacgg gacctgacgc tccgcctggc gcgattcctc cttctcctcc tcctcagcca 2400
   aaggcgccgg tcgctcctgc cttcgagagc gagcttcgga aaggaggatg aacgagggtg 2460
   aacagggcgg ccggggaccc gaagggggga tcccttcagt ccttcgccat tcactttccc 2520
   ctctagcctc acacccccct ttatattctg cttcacgtgg ggtgcggggc aggagggagg 2580
   gggagagcgc aggaagggga ggaaaagcga gaaatgccca ccttctccgg ccaactccga 2640
   agccgcctca gccccctcct cccgccgcgt cggcggccgc tgcaaagccc tcccggccgc 2700
   tctgcacgga ggcagcgcca agcaaatgtc gccttcccct cgccaccccc ggggccgccg 2760
   ccgccgccag cgagccccga gctcagtgtc gctcaaacct cccctcccgg ccccgctcgg 2820
   ccctcccccc gcgccccgca gaccctttca aaccccctgg gctcgcggcg gctgctcggt 2880
   gaggaaacgc tggccgcggc tggggccggg gcgacgcggc tgcgagcggg gcggcggcga 2940
   ggcgcccggt acggtgagcc cagcaacgag gcgcgggcgg gcgcgcagag acgccgggtc 3000
   ctcgcggagc acggccgggg cgcggcggcg gcggtggcag cgggagccca gggaccgctc 3060
   catcactggc agccatggag cccgagcatt ttcctccctc agggcaggcg cctcgactcg 3120
   gcacacgtcc tcggggagga aggggccggc acccgagcct ctccggaggg cggcgggacg 3180
   tgcgctaggg cggagggtgc gaccctctct tatcctcttc ctccgcagcc ggctcttcct 3240
   caccccgctg ctcccgcgac cgggcgggag agggccgcag gcggcggcgg ctcctttctc 3300
   cgcggcgttg gtggccagac gggctggtgc agcgactgca gccgacgccg ttcaaaggaa 3360
   cgaggtgggg ttggtcggtg tcggcaaacg gggcggggga gtgagtgggt gccgagggag 3420
   ggtgtcgggc ggggaggggg ctgtggtggt tgttactagt gctcttcttc ggctcccccg 3480
   gctccaccgt atcccccagt ctctcgcggc atcgggaggg gagggaccag cgcggagcgc 3540
   aggaggagga ggggcgaccg ccggcgagag gaggcggtgg tggctggggg gagggggcag 3600
   gcggaggagg aggagggaag atggcgtctg cgcatgcgcc tcggcgccgc agacataaag 3660
   ccgggtctgg cggggcagga ggcggcgggt cttcgggcct aggcgagcgc caggtctccc 3720
   gacggaaatt accgaaggct ggcagaggcg ccgaagccgg gagtggtgga gccggagggg 3780
   atagtggggg tggggaattc cggccgaagg agcgaatccg ggtcggactg cgtgattcgg 3840
   cccggtgggg gcgggcaggg tgcgcgcctg ccgcgcgccc aggttaggtg tgtccgctct 3900
   tttcacactc gcggccggcg cgcgcgggca cgcggcagag gagggggcgc ggcgcggaag 3960
   ggccggcgga ggcggcggga gcgccggcgc ttggtgtgcg cgccttgggt ggtgtccccc 4020
   gcctgcggaa tgggtaatga ggcttttctc cgcagcaaca gcatcacgag tttcagttcc 4080
   caggagcccg ccgccgcggc tgctgccgct gccacgacat gagtcaggct ttcttccctg 4140
   gcgcgtggcg gaaacctcct ccccctcgcc gcacctgggc tcgcccccgg cccttgcctc 4200
   ctcgggggat gccctagggc cgggagctgt ggggtctatg gaaggccggt ggctcccggt 4260
   gataggtgtt gt 4272
<210> 20
   <211> 21
   <212> DNA
   <213> Artificial Sequence
<220>
   <223> primer
<400> 20
   gtagagcgtt tagattgatt c 21
<210> 21
   <211> 19
   <212> DNA
   <213> Artificial Sequence
<220>
   <223> primer
<400> 21
   ctaacgttac aaaaccccg 19
<210> 22
   <211> 23
   <212> DNA
   <213> Artificial Sequence
<220>
   <223> primer
<400> 22
   cgtcgtaaat ttcgaagaga tac 23
<210> 23
   <211> 25
   <212> DNA
   <213> Artificial Sequence
<220>
   <223> primer
<400> 23
   aaataaaaac gacaaaaaca acgtt 25
<210> 24
   <211> 25
   <212> DNA
   <213> Artificial Sequence
<220>
   <223> primer
<400> 24
   tttgttgtat tgagagtaga ggtgt 25
<210> 25
   <211> 26
   <212> DNA
   <213> Artificial Sequence
<220>
   <223> primer
<400> 25
   aacattaaca ctaaaaaaat aacaca 26
<210> 26
   <211> 33
   <212> DNA
   <213> Artificial Sequence
<220>
   <223> primer
<400> 26
   gggatattaa gtggagttat tttggtttta gtt 33
<210> 27
   <211> 21
   <212> DNA
   <213> Artificial Sequence
<220>
   <223> primer
<400> 27
   ccctcccaac atccttccta a 21
<210> 28
   <211> 255
   <212> DNA
   <213> Homo sapiens
<400> 28
   gggtttagcc acaggcttgt ccagggcgct cactcctcgc caggagcggc gccggtgctt      60
   tggttacacc ccgagaggcg cccggcacta cggtttcctt gtgcgctgct ggaattttca     120
   gggttggagc gccggccgcg taggggtggc agtgaggccg cgcagtgagg ggacggactc     180
   gcccgaggtc gcagagcgtt cagattgact cgacaggctg gggggcgggg ccggggtccc     240
   cacgcggagt ccggg                                                      255
<210> 29
   <211> 146
   <212> DNA
   <213> Homo sapiens
<400> 29
   gtataccctg gaacctgtga cagcgtcgca aatcccgaag agacacgaaa atagggcgaa      60
   atagaaaatc ccagccatgg ttcgccggtg ccaacgctgc tcctgccgct tctatcccag     120
   tggaataaat gcttaagtta ggagag                                          146
<210> 30
   <211> 255
   <212> DNA
   <213> Homo sapiens
<400> 30
   gaggttcagg ctttgtggag gcggaggctg tggtcccgac gtagaagatg ctgcaggggg      60
   cggcggctga agcagcgacg gaggcggaaa atcctcggaa gatgtggcat tactaccgac     120
   gccggtacct gctgcattga gagcagaggc gctgccacta ccgctgcccc ttcgagggaa     180
   cattgccggg tgcgccatct tcctagcgct aatgtctgca gcggcggcgg ccgcggcggt     240
   ggactcaggc ggctg                                                      255
<210> 31
   <211> 79
   <212> DNA
   <213> Homo sapiens
<400> 31
   cagagcgttc agattgactc gacaggctgg ggggcggggc cggggtcccc acgcggagtc 60
   cggggccctg caacgttag 79
<210> 32
   <211> 94
   <212> DNA
   <213> Homo sapiens
<400> 32
   cgtcgcaaat cccgaagaga cacgaaaata gggcgaaata gaaaatccca gccatggttc 60
   gccggtgcca acgctgctcc tgccgcttct atcc 94
<210> 33
   <211> 92
   <212> DNA
   <213> Homo sapiens
<400> 33
   tacctgctgc attgagagca gaggcgctgc cactaccgct gccccttcga gggaacattg 60
   ccgggtgcgc catcttccta gcgctaatgt ct 92
<210> 34
   <211>
   <212> DNA
   <213> Artificial Sequence
<220>
   <223> primer
<400> 34
   tatttgttgt attgagagta gaggc 25
<210> 35
   <211>
   <212> DNA
   <213> Artificial Sequence
<220>
   <223> primer
<400> 35
   aaacattaac gctaaaaaaa taacg 25

## Description

### TECHNICAL FIELD

The present invention relates to a method for determining the presence or absence of cancer cells derived from hepatocellular carcinoma in a biological sample. More specifically, the present invention relates to a method for determining the presence or absence of cancer cells in a biological sample on the basis of the result of analysis of methylation status of a CpG site in a promoter region of certain genes in DNA extracted from the biological sample. The present invention also relates to a determination marker and a determination kit used for the method.

### BACKGROUND ART

Hepatocellular carcinoma is a type of primary liver cancer and is a malignant tumour originated from hepatocytes. Hepatocellular carcinoma is almost inactive in the initial stage. However, when subjective symptoms such as abdominal lumps, sudden abdominal pain or jaundice are manifested, the disease has already been developed to severe stages in most cases. Hepatocellular carcinoma is known to often develop in patients with chronic hepatitis or hepatic cirrhosis resulting from Hepatitis B virus (HBV) or Hepatitis C virus (HCV). Thus early detection of hepatocellular carcinoma has been sought by identifying the patients as a high-risk group and proactively examining the patients.

Examination for hepatocellular carcinoma includes measurement of tumor markers in blood. The tumor markers currently used include AFP (α-fetoprotein), PIVKA-II (protein induced by vitamin-K absence II) and the like. The examination of the tumor markers is used as screening of patients with hepatocellular carcinoma; however the markers have insufficient sensitivity and specificity. For example, the examination of AFP may not result in an abnormal value for hepatocellular carcinoma with relatively small size and the examination of PIVKA-II may provide false positive results for subjects with vitamin K deficiency or subjects taking warfarin. Therefore definitive diagnosis of hepatocellular carcinoma is made by, in addition to the examination of tumor markers, ultrasonic examination and examination by imaging such as CT and MRI.

Meanwhile, new detection methods for cancer have been recently studied which are based on genetic information. The detection methods for cancer include, for example, ones based on information on methylation of genes. The methods use markers which are CpG sites (5'-(CG)-3') in certain genes and cancer is detected on the basis of information obtained by analyzing methylation status of the markers.

Methods for detecting cancer utilizing methylation analyses of genes have been studied for hepatocellular carcinoma. For example, the publication by Ying-Bing Deng et al. discloses that genes such as BCAN, ZNF141 and DUSP4 are not methylated in normal liver tissues or non-cancerous liver tissues while the genes are highly methylated in hepatocellular carcinoma caused by HCV infection (see Non-Patent Literature 1). The publication by Ran Tao et al. also discloses that genes such as WNK2, EMLIN2 and TLX3 are not methylated in non-cancerous liver tissues while the genes are highly methylated in hepatocellular carcinoma caused by HBV infection (see Non-Patent Literature 2).

### Citation List

### Non-Patent Literature

Non-Patent Literature 1: Ying-Bing Deng et al., Cancer Sci, June 2010, vol. 101, no. 6, 1501-1510
Non-Patent Literature 2: Ran Tao et al., ProS ONE, May 2011, Vol. 6, Issue. 5, e19862

### SUMMARY OF INVENTION

Some genes which are abnormally methylated in hepatocellular carcinoma have been reported as described above. The detection sensitivity of hepatocellular carcinoma utilizing methylation analyses of genes can be improved by increasing the number of genes which can be used as detection markers. However, there are only a small number of genes that can be used as markers for detection of hepatocellular carcinoma. Therefore there is a need for further development of novel markers for detection of hepatocellular carcinoma utilizing methylation analyses of genes.

Accordingly an object of the present invention is to provide a method for determining the presence or absence of a cancer cell derived from hepatocellular carcinoma by identifying such a novel marker and analyzing methylation status of the marker. Another object of the present invention is to provide a kit suitably used for the method.

The present inventors have analyzed the methylation status of DNAs from cancerous tissues of hepatocellular carcinoma, normal liver tissues and tissues of chronic hepatitis / hepatic cirrhosis and identified novel markers which are genetic regions specifically methylated in cancerous tissues. The present inventors have found that cancerous tissues from hepatocellular carcinoma can be clearly discriminated with high accuracy from normal liver tissues or from tissues of chronic hepatitis / hepatic cirrhosis based on the result obtained by analyzing the methylation status of certain base sequences in the genetic regions, thereby achieving the present invention.

Thus the present invention provides a method for determining the presence or absence of a cancer cell derived from hepatocellular carcinoma comprising the steps of:
extracting DNA from a biological sample collected from a subject;
analyzing the methylation status of a CpG site in a promoter region of at least one gene selected from ACTG1 (Actin, gamma 1), EPHA4 (EPH receptor A4) and TSC22D1 (TSC22 domain family, member 1) in the DNA obtained from the extraction step; and
determining the presence or absence of a cancer cell derived from hepatocellular carcinoma in the biological sample based on an analysis result obtained from the analyzing step.

The present invention also provides a marker for determining the presence or absence of a cancer cell derived from hepatocellular carcinoma by methylation analysis, which is at least one CpG site selected from the CpG sites located in the promoter regions of ACTG1, EPHA4 and TSC22D1 genes.

The present invention further provides a kit for determining the presence or absence of a cancer cell derived from hepatocellular carcinoma, comprising a primer set for analysis of methylation status of at least one CpG site selected from CpG sites located in promoter regions of ACTG 1, EPHA4 and TSC22D1 genes.

The present invention can provide a novel marker which allows determination of the presence or absence of a cancer cell derived from hepatocellular carcinoma. The present invention can also provide a method and a kit for determining the presence or absence of a cancer cell derived from hepatocellular carcinoma in a biological sample collected from a subject by analyzing methylation status of the marker. With the present invention, an index that may allow determination on whether or not a subject has developed hepatocellular carcinoma can be provided to physicians and the like at clinical sites.

### BRIEF DESCRIPTION OF DRAWINGS

Figs. 1A, 1B and 1C are views illustrating the principle of MassARRAY^{®} analysis;
Fig. 2 is a graph indicating the methylation rate of the promoter region of the ACTG1 gene in genomic DNAs extracted from various clinical specimens;
Fig. 3 is a graph indicating the methylation rate of the promoter region of the EPHA4 gene in genomic DNAs extracted from various clinical specimens;
Fig. 4 is a graph indicating the methylation rate of the promoter region of the TSC22D1 gene in genomic DNAs extracted from various clinical specimens;
Fig. 5 is a graph indicating the methylation rate of the DUSP4 gene in genomic DNAs extracted from various clinical specimens;
Fig. 6 is a graph indicating the methylation rate of the ZNF141 gene in genomic DNAs extracted from various clinical specimens;
Fig. 7 is a graph indicating the methylation rate of the SFRP1 gene in genomic DNAs extracted from various clinical specimens;
Fig. 8 is a graph indicating the methylation rate of the GSTP1 gene in genomic DNAs extracted from various clinical specimens;
Fig. 9 is an image showing the results of amplification of DNAs extracted from normal liver tissues, hepatic cirrhosis tissues and hepatocellular carcinoma tissues by a methylation specific PCR (MSP) method using primer sets for ACTG1, EPHA4 and TSC22D1;
Fig. 10 is a graph indicating the results obtained by amplifying DNAs extracted from plasma of healthy subjects and patients with hepatocellular carcinoma by the MSP method using the primer set for TSC22D1 and quantifying the amount of the resulting amplification products by means of gel electrophoresis and image processing;
Fig. 11 is a graph indicating the results obtained by amplifying DNAs extracted from plasma of healthy subjects and patients with hepatocellular carcinoma by the MSP method using the primer set for EPHA4 and quantifying the amount of the resulting amplification products by means of gel electrophoresis and image processing; and
Fig. 12 is a graph indicating the results obtained by amplifying DNAs extracted from plasma of healthy subjects and patients with hepatocellular carcinoma by the MSP method using a primer set for accuracy control and quantifying the amount of the resulting amplification products by means of gel electrophoresis and image processing.

### BEST MODE FOR CARRYING OUT THE INVENTION

In the method for determining the presence or absence of a cancer cell derived from hepatocellular carcinoma of the present invention (hereinafter also referred to as "determination method"), DNA is first extracted from a biological sample collected from a subject.

In the present embodiments, the biological sample is not particularly limited as far as it is a biological sample containing DNA of a subject and is preferably a sample containing a genomic DNA, such as a clinical specimen. The clinical specimen may include, for example, blood, serum, plasma, lymph fluid, ascetic fluid, bone marrow aspirate, urine, nipple discharge, tissues obtained by operations or biopsies and the like. The biological sample may also be a culture medium obtained by culturing cells or tissues collected from a subject.

DNA can be extracted from a biological sample according to well known extraction methods. Extraction of DNA can be carried out, for example, by mixing a biological sample with a treatment solution containing a surfactant for solubilization of cells or tissues (e.g. sodium cholate, sodium dodecyl sulfate etc.), and subjecting the resulting mixture to physical procedure (stirring, homogenization, ultrasonication etc.) to release DNA contained in the biological sample into the mixture. In this case, it is preferable to centrifuge the mixture of the biological sample and the treatment solution to precipitate cell debris and use the supernatant containing the released DNA to the next step of analyzing described hereinbelow. The obtained supernatant may be purified according to well known methods. DNA can also be extracted and purified from a biological sample by using commercially available kits.

The extraction step preferably further comprises the step of fragmenting the extracted DNA. By fragmenting DNA to have an appropriate length, methylated DNA immunoprecipitation (MeDIP) and non-methylated cytosine conversion as described hereinbelow can be effectively carried out.

Fragmentation of DNA may be carried out by ultrasonication, alkaline treatment, restriction enzyme treatment and the like. When DNA is fragmented by alkaline treatment, a sodium hydroxide solution may be added to a DNA solution to the final concentration of 0.1N to 1.0N and the mixture may be incubated at 10°C to 40°C for 5 to 15 minutes to fragment the DNA. In case of DNA fragmentation by means of the restriction enzyme treatment, the restriction enzyme used may appropriately be selected based on the base sequence of DNA, that may be *Mse*I or *Bam*HI, for example.

According to the present determination method, the methylation status of a CpG site in a promoter region of at least one gene selected from ACTG1, EPHA4 and TSC22D1 in DNA obtained in the extraction step is analyzed.

As used herein, the term "CpG site" means a site of a sequence in which cytosine (C) and guanine (G) are adjacent in this order from 5' to 3' in a base sequence. The letter "p" in "CpG" represents a phosphodiester bond between cytosine and guanine.

As used herein, to "analyze the methylation status" means to analyze the presence or absence of methylation of a CpG site located in a promoter region of at least one gene selected from ACTG 1, EPHA4 and TSC22D1 or analyze the methylation frequency in the promoter region.

The base sequences *per se* of the promoter regions of ACTG1, EPHA4 and TSC22D1 genes are well known in the art. The base sequences can be obtained from well-known databases such as the one provided by the National Center for Biotechnology Information (NCBI) of the US National Library of Medicine (http://www.ncbi.nlm.nih.gov/). The ID numbers of the above genes are shown in Table 1. The base sequences of the promoter regions of the genes are represented by SEQ ID NOs: 17 to 19, respectively.

**Table 1**

| **Gene symbol** | Unigene ID | Entrez Gene ID | Transcript ID | **SEQ ID NO:** |
|---|---|---|---|---|
| ACTG1 | Hs.514581 | 71 | NM_001614.3 | 17 |
| EPHA4 | Hs.371218 | 2043 | NM_004438.3 | 18 |
| TSC22D1 | Hs.436383 | 8848 | NM_183422.3 | 19 |

In particular embodiments of the present invention, the analyzing step may be the step of analyzing the presence or absence of methylation of at least one CpG site among CpG sites located in a promoter region of at least one gene selected from ACTG 1, EPHA4 and TSC22D1 genes. The term "presence or absence of methylation" means whether or not cytosine in a CpG site located in the promoter region is methylated. In particular embodiments, one CpG site may be analyzed; however, more than one CpG site is preferably analyzed for the presence or absence of methylation in order to improve the determination accuracy in the following determination step. More than one CpG site may be selected from a promoter region of one gene or from each of promoter regions of more than one gene.

In another embodiment of the present invention, the analyzing step may be the step of analyzing the methylation frequency in a promoter region of at least one gene selected from ACTG 1, EPHA4 and TSC22D1 genes. The term "methylation frequency" means a ratio of the number of methylated CpG site(s) relative to the number of CpG site(s) located in the promoter region. In the embodiment, the target for analysis may be the whole promoter region or a part thereof including at least one CpG site. The target for analysis may contain only one CpG site; however it is preferable that the target for analysis contains more than one CpG site in order to improve the determination accuracy in the following determination step. The target for analysis may be selected from any one promoter region among the above genes or from promoter regions of more than one gene.

The positions and numbers of CpG sites located in the promoter regions of ACTG 1, EPHA4 and TSC22D1 genes are already known. Thus the number of methylated CpG site(s) itself in the promoter regions can also be used as the methylation frequency.

The methylation frequency may be "methylation rate" obtained by analyzing methylation status of a CpG site in DNA with mass spectrometry such as MassARRAY^{®} as described hereinbelow. MassARRAY^{®} allows calculation of the methylation rate based on the ratio between the area of a peak derived from a methylated DNA fragment and the area of a peak derived from a non-methylated DNA fragment obtained after measurement of the DNA fragments.

In particular embodiments of the present invention, the target for analysis may be any CpG site(s) or region(s) including the CpG site(s) in the promoter regions of ACTG1, EPHA4 and TSC22D1 genes without particular limitation. The positions and numbers of CpG sites located in the promoter regions of the genes are already known. Thus the target CpG site or region may be appropriately selected by a person skilled in the art based on the routine experiments according to the well known analysis methods described hereinbelow.

Various methods are well known in the art as to the method for analyzing methylation status. According to the present method, it is not specifically limited as to which analysis method is used; however, the analysis method preferably comprises differentiating methylated DNA from non-methylated DNA, amplifying DNA and detecting methylated DNA and/or non-methylated DNA.

The step of differentiating methylated DNA from non-methylated DNA may include the step of carrying out methylation sensitive restriction enzyme treatment, a MeDIP method, non-methylated cytosine converting treatment and the like.

The step of amplifying DNA may include the step of carrying out PCR, quantitative PCR, IVT (*in vitro* transcription) amplification, SPIA™ amplification and the like methods.

The step of detecting methylated DNA and/or non-methylated DNA may include the step of carrying out electrophoresis, sequence analysis, microarray analysis, mass spectrometry, Southern hybridization and the like.

The MeDIP method is a method in which methylated DNA in a biological sample is concentrated by immunoprecipitation using an anti-methylated cytosine antibody or an anti-methylated cytidine antibody, or an antibody which specifically recognizes a methylated DNA-binding protein. In particular embodiments of the present invention, the analyzing step may be the step of concentrating methylated DNA in DNA obtained in the extraction step by the MeDIP method and analyzing methylation status of the obtained methylated DNA. The methylated DNA concentrated by the MeDIP method may be amplified by e.g. IVT amplification and methylation status of the obtained amplified product may be analyzed by using a microarray. These analysis procedures are referred to as the MeDIP on chip method.

The non-methylated cytosine converting treatment is the one in which DNA extracted from a biological sample is subjected to reaction with a non-methylated cytosine conversion agent so as to convert non-methylated cytosine(s) in the DNA to a different base (uracil, thymine, adenine or guanine). In this context, the non-methylated cytosine conversion agent is a substance which can react with DNA and convert a non-methylated cytosine in the DNA to a different base (uracil, thymine, adenine or guanine). The non-methylated cytosine conversion agent suitably used may be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status of a CpG site in DNA is converted to the difference in a base sequence (C and U) by the non-methylated cytosine converting treatment using bisulfite. The non-methylated cytosine converting treatment using bisulfite is referred to as bisulfite treatment.

When the bisulfite treatment is carried out, the amount (concentration) of bisulfite added is not specifically limited so long as it can sufficiently convert non-methylated cytosine(s) in DNA, and corresponds to, for example, 1M or higher, preferably 1M to 15M, more preferably 3M to 10M as the final concentration in a solution containing DNA. The incubation conditions (temperature and time) after addition of bisulfite may be appropriately selected according to the amount added of bisulfite, and for example, when bisulfite is added at a final concentration of 6M, the incubation is carried out at 50°C to 80°C for 10 minutes to 90 minutes.

Methylation status of CpG sites in DNA can be analyzed by analyzing the sequence of DNA after bisulfite treatment and detecting the difference in the base sequence from the original sequence. This process is referred to as a bisulfite sequencing method.

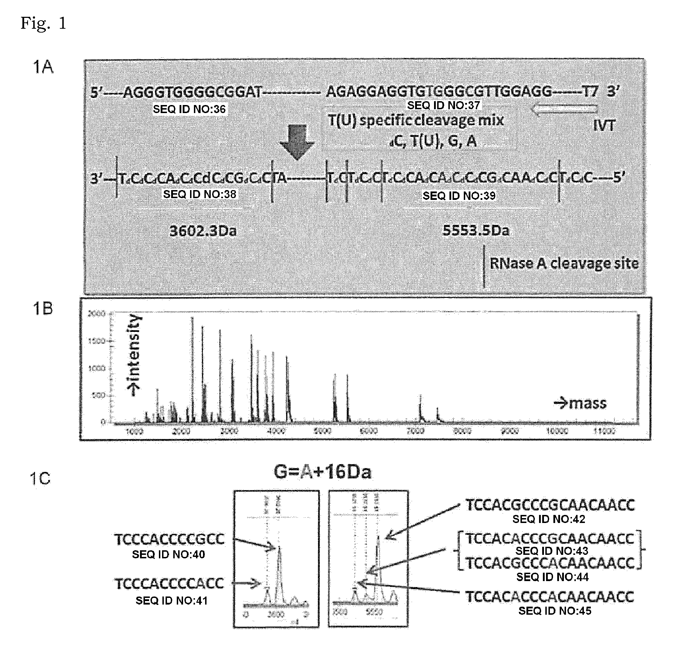

Methylation status of CpG sites can be alternatively analyzed by mass spectrometry. Specifically, a DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is digested with RNase A and the difference in the mass (16 Da) due to difference between G and A between the obtained digested fragments is detected on a MALDI-TOF (matrix assisted laser desorption/ionization time-of-flight) mass spectrometer to analyze methylation status of DNA. This method is referred to as MassARRAY^{®} analysis (see Fig. 1).

As shown in Fig. 1A, it is known that the cleavage site in IVT products by RNase A is between an arbitrary base and the adjacent uracil (U) or thymine (T). Thus the base sequence and mass of the IVT product cleaved by RNase A may be predicted based on the base sequence of the template DNA. Accordingly the peaks obtained in MassARRAY^{®} can be allocated to the portions of the base sequences in the template DNA from which the peaks are originated. For example, when one CpG site is methylated in a template DNA fragment, a peak obtained in MassARRAY^{®} shifts to the side with an increased mass for 16 Da (see the left panel in Fig. 1C). When a DNA fragment containing more than one CpG site is analyzed for example, the DNA fragment having two methylated CpG sites shows a shift of 32 Da (see the right panel in Fig. 1C) and the DNA fragment having three methylated CpG sites shows a shift of 48 Da.

In mass spectrometry such as MassARRAY^{®}, the methylation rate of the analyzed DNA fragments can be calculated. The calculation is exemplified by referring to the left panel shown in Fig. 1C. When the ratio between the area of the peak (left) of a non-methylated DNA fragment and the area of the peak (right) of a methylated DNA fragment is 1:3, the methylation rate of the DNA fragments having the sequence is 75% (3/(1+3) = 0.75). The methylation rate is theoretically 100% for a DNA fragment in which all CpG site(s) is methylated and 0% for a DNA fragment without any methylated CpG site.

Methylation status of CpG sites can be analyzed by a methylation specific PCR (MSP) method. The MSP method is a method in which methylation status of CpG sites (presence or absence of methylation) is analyzed by amplifying DNA after bisulfite treatment by PCR using a primer set described hereinafter and determining the presence or absence of a PCR product.

The MSP method utilizes a primer set which can amplify a base sequence having a CpG site to be analyzed that is methylated (i.e. cytosine is not converted to uracil) but cannot amplify a base sequence having a CpG site that is not methylated (i.e. cytosine is converted to uracil). According to the MSP method using such a primer set, the presence of a PCR product indicates methylation of the CpG site analyzed.

The MSP method may also be carried out by using a primer set which cannot amplify a base sequence having cytosine in a CpG site to be analyzed that is not converted to uracil but can amplify a base sequence having cytosine in a CpG site that is converted to uracil. In this case, absence of a PCR product indicates methylation of the CpG site analyzed.

Each primer in the primer set used for the MSP method may be appropriately designed by a person skilled in the art depending on the base sequence including a CpG site to be analyzed, and it is preferably designed so as to contain cytosine of the CpG site to be analyzed at the 3' end or in the vicinity thereof of the primer.

Methylation status of CpG sites may alternatively be analyzed with a microarray. In this case, the microarray for analysis may be prepared by immobilizing a nucleic probe complementary to the base sequence of a promoter region of ACTG1, EPHA4 or TSC22D1 gene on a substrate. The microarray can be prepared according to well known methods in the art.

In the analysis using a microarray, DNA extracted from a biological sample is preferably labelled with a labelling substance well-known in the art. Thus, the present determination method preferably further comprises the step of labelling the extracted DNA. The labelling step is advantageously carried out after the DNA amplifying step because all DNA in the biological sample may be labelled. The labelling substance may include fluorescence substances, haptens such as biotin, radioactive substances and the like. The fluorescence substances may include Cy3, Cy5, FITC, Alexa Fluor™ and the like.

Labelling of DNA facilitates measurement of a signal from a probe on the microarray. A method for labelling DNA with the labelling substance is well known in the art.

The above signal may be any suitable signal depending on the type of microarrays. For example, the signal may be an electric signal generated when a DNA fragment hybridizes to a probe on the microarray, or a fluorescence or luminescence signal generated from a labelling substance when DNA to be analyzed is labelled as described above.

Detection of a signal can be carried out by using a scanner comprised in a conventional microarray analyzer. The scanner may be, for example, GeneChip^{®} Scanner3000 7G (Affymetrix), Illumina^{®} BeadArray Reader (Illumina) and the like.

In the present method, the presence or absence of a cancer cell derived from hepatocellular carcinoma in the biological sample is determined based on the analysis result obtained in the analyzing step.

In the embodiments of the present invention, when the analysis result from the analyzing step shows the presence of a methylated CpG site, it may be determined that the biological sample collected from the subject contains a cancer cell. When the result from the analyzing step shows absence of a methylated CpG site on the contrary, it may be determined that the biological sample does not contain a cancer cell.

The above determination may be based on the analysis result of one CpG site. However in order to improve the determination accuracy, it is preferable to make a determination based on the analysis result of more than one CpG site.

In another embodiment of the present invention, when the methylation frequency obtained in the analyzing step is higher than a certain threshold, it may be determined that the biological sample collected from the subject contains a cancer cell. When the methylation frequency obtained is lower than a certain threshold on the contrary, it may be determined that the biological sample collected from the subject does not contain a cancer cell.

The threshold is not particularly limited and may be empirically set based on accumulated data on various biological samples. The threshold may alternatively be set as follows. First, methylation frequency is analyzed for DNA extracted respectively from a biological sample which is confirmed to be devoid of cancer cells derived from hepatocellular carcinoma (normal liver tissue or normal hepatocyte) and a biological sample containing the cancer cell. Next, based on the obtained analysis results, a threshold is set within a range that is higher than the methylation frequency of the biological sample devoid of cancer cells and lower than that of the biological sample containing the cancer cell. Preferably, the threshold is set as a value which can highly accurately differentiate between the biological sample devoid of cancer cells and the biological sample containing the cancer cell.

In another embodiment of the present invention, the method may further comprise the diagnosis step of making definitive diagnosis on the subject from which the biological sample has been collected based on a determination result obtained from the determination step. Thus the scope of the present invention encompasses the method for diagnosis of hepatocellular carcinoma (hereinafter referred to as "diagnosis method"). More specifically the diagnosis method of the present invention comprises the following steps:
extracting DNA from a biological sample collected from a subject;
analyzing methylation status of a CpG site in a promoter region of at least one gene selected from ACTG1, EPHA4 and TSC22D1 in the DNA obtained from the extraction step;
determining the presence or absence of a cancer cell derived from hepatocellular carcinoma in the biological sample based on an analysis result obtained from the analyzing step; and
carrying out an examination for definitive diagnosis on the subject from which the biological sample has been collected depending on a determination result obtained from the determination step.

In the diagnosis step when the biological sample is determined to contain a cancer cell in the determination step, an examination for definitive diagnosis is carried out on the subject from which the biological sample has been collected. When the biological sample is determined to be devoid of cancer cells on the contrary, an examination for definitive diagnosis is not carried out. The examination for definitive diagnosis may be carried out in a well-known manner that is used for definitive diagnosis of hepatocellular carcinoma in the art without limitation and may include, for example, diagnostic imaging including ultrasound examination, radiography, computed tomography (CT), nuclear magnetic resonance imaging (MRI) or positron emission tomography (PET) and biopsy. In particular embodiments of the present invention, when a subject is found to have hepatocellular carcinoma by means of at least one of these manners, the subject can be definitively diagnosed as hepatocellular carcinoma.

The scope of the present invention also encompasses a marker for determining the presence or absence of a cancer cell derived from hepatocellular carcinoma by methylation analysis (hereinafter also referred to as "determination marker"). The determination marker of the present invention is at least one CpG site selected from CpG sites located in promoter regions of ACTG1, EPHA4 and TSC22D1 genes.

In the embodiments of the present invention, the DNA extracted from a biological sample is analyzed for methylation status of the determination marker according to the analysis method as described above and the biological sample can be determined whether it contains or not a cancer cell derived from hepatocellular carcinoma.

The scope of the present invention also encompasses a kit for determining the presence or absence of a cancer cell derived from hepatocellular carcinoma (hereinafter also referred to as "determination kit"). The determination kit of the present invention comprises a primer set for analysis of methylation status of at least one CpG site selected from CpG sites located in promoter regions of ACTG1, EPHA4 and TSC22D1 genes.

In the embodiments of the present invention, the primer set in the determination kit may be a primer set for analysis of methylation status of CpG sites according to mass spectrometry such as MassARRAY^{®} or an analysis method involving PCR amplification such as the MSP method, the bisulfite sequencing method.

The base sequence of the primers in the primer set may be appropriately selected based on the base sequences in the promoter regions. The primer set used for mass spectrometry such as MassARRAY^{®} for example may be a primer set of primers respectively having base sequences SEQ ID NOs: 1 and 2, a primer set of primers respectively having base sequences SEQ ID NOs: 3 and 4 and a primer set of primers respectively having base sequences SEQ ID NOs: 5 and 6. The primer set used for the MSP method may be a primer set of primers respectively having base sequences SEQ ID NOs: 20 and 21, a primer set of primers respectively having base sequences SEQ ID NOs: 22 and 23 or a primer set of primers respectively having base sequences SEQ ID NOs: 24 and 25.

The present invention is more specifically described hereinafter by way of Examples which do not limit the present invention.

### Examples

### Example 1: Identification of novel markers from genomic DNA of hepatocellular carcinoma tissues

### (1-1) Biological samples

In the present Example, the biological samples used were clinical specimens obtained from patients by hepatectomy. The clinical specimens contained cancerous tissues of hepatocellular carcinoma (100 specimens), normal liver tissues (3 specimens) and chronic hepatitis / hepatic cirrhosis tissues (39 specimens). The tissues were immediately frozen with liquid nitrogen after collection and stored at -80°C prior to use.

### (1-2) DNA extraction and bisulfite treatment

Genomic DNA was extracted with a QIAamp DNA Mini Kit (QIAGEN) from the above tissues. The obtained DNA (500 ng) was subjected to bisulfite treatment with an EZ DNA Methylation Kit (Zymo Research) and the genomic DNA after treatment was dissolved in sterilized distilled water (10 µl). The genomic DNA contained in the obtained DNA solution (4 µl) was fragmented with a Bioruptor (COSMO BIO).

### (1-3) Identification of novel markers

The fragmented genomic DNA was subjected to Infinium Methylation Assay using an Infinium HumanMethylation27 BeadChip (Illumina) in order to search novel markers which are specifically methylated in cancerous tissues of hepatocellular carcinoma. The specific procedures carried out followed the manual attached to the chip.

As a result, promoter regions of ACTG1, EPHA4 and TSC22D1 genes were identified as markers which are specifically methylated in cancerous tissues of hepatocellular carcinoma. These markers may be referred to as the present markers hereinbelow.

### Example 2: Assessment of performance of present markers by mass spectrometry

### (2-1) Biological samples

In the present Example, the biological samples used were clinical specimens obtained from patients different from those used in Example 1 by hepatectomy. The clinical specimens contained normal liver tissues (2 specimens), hepatic cirrhosis tissues (2 specimens) and cancerous tissues of hepatocellular carcinoma (6 specimens). The tissues were immediately frozen with liquid nitrogen after collection and stored at -80°C prior to use.

### (2-2) Preparation of measurement samples and control samples (i) Extraction of genomic DNA

Genomic DNA was extracted from the above tissues with a QIAamp DNA Mini Kit (QIAGEN). The genomic DNA contained in the obtained DNA solution was fragmented with a Bioruptor (COSMO BIO).

For preparation of a calibration curve for mass spectrometry, the control genomic DNA used was genomic DNA from human peripheral blood lymphocytes. The genomic DNA from human peripheral blood lymphocytes was amplified with a GenomiPhi v2DNA amplification kit (GE Healthcare Life Sciences). The obtained amplification product consisted of non-methylated DNA. The amplification product was fragmented with a Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA). The solution of 0% methylated DNA and the solution of 100% methylated DNA were mixed at certain proportions to obtain solutions of 25%, 50% and 75% methylated DNA.

### (ii) Bisulfite treatment

The respective DNA fragments (1 µg) obtained as above were subjected to bisulfite treatment with an EZ DNA Methylation Kit (Zymo Research) and the treated genomic DNA was dissolved in sterilized distilled water (160 µl).

### (iii) Amplification by PCR and IVT

Methylated cytosine and uracil in the DNA fragments after bisulfite treatment were converted respectively to guanine and adenine by PCR and IVT amplification.

It is verified by MassARRAY® analysis using control samples as described hereinbelow that the primer sets used for PCR can amplify both methylated DNA and non-methylated DNA without bias. The sequences of the primer sets for the present markers are shown in Table 2. The base sequences which can be analyzed with the primer sets shown in Table 5 in the promoter regions in ACTG1, EPHA4 and TSC22D1 genes are shown in SEQ ID NOs: 28 to 30, respectively.

**Table 2**

| **Marker** | **Forward primer** | **SEQ ID NO:** | **Reverse primer** | **SEQ ID NO:** |
|---|---|---|---|---|
| ACTG1 | GGGTTTAGTTATAGGTTTGTTT | 1 | ACCCTAACTCRCTATTATCACT | 2 |
| EPHA4 | GTATATTTTGGAATTTGTGATAG | 3 | CTCTCCTAACTTAAACATTTATTT | 4 |
| TSC22D1 | GAGGTTTAGGTTTTGTGGAG | 5 | TACCCAACACAATACACCAA | 6 |

The following tag sequence and T7 promoter sequence were respectively added to the 5'-terminals of the forward primers and reverse primers in the above primer sets for IVT reaction.
- Tag sequence: AGGAAGAGAG (SEQ ID NO: 7)
- T7 promoter sequence: CAGTAATACGACTCACTATAGGGAGAAGGCT (SEQ ID NO: 8)

PCR reaction solution was prepared by mixing the following reagents:

| | |
|---|---|
| 10x Hot Star buffer (QIAGEN) | 0.5 µL |
| 25 mM dNTP mix | 0.04 µL |
| Hot Star Taq (5 U/µL) (QIAGEN) | 0.04 µL |
| Primer mix | 2.0 µL |
| DNA solution | 1.0 µL |
| Water | 1.42 uL |
| Total | 5 µL |

PCR reaction was carried out on the reaction solution under the following conditions:
1 cycle of 94°C for 15 minutes;
45 cycles of 94°C for 20 seconds, 52°C for 30 seconds and 72°C for 1 minute, and 72°C for 3 minutes.

The obtained PCR products were dephosphorylated with SAP (Shrimp Alkaline Phosphatase) in a MassCLEAVE™ Reagent kit (SEQUENOM). The following reaction solution prepared with the kit was then added.

| | |
|---|---|
| 5x T7 R&DNA polymerase buffer | 0.89 µL |
| T Cleavage mix | 0.24 µL |
| 100 mM DTT | 0.22 µL |
| T7 R&DNA polymerase | 0.44 µL |
| RNase A | 0.06 µL |
| RNase-free water | 3.15 µL |
| Total | 5 µL |

The obtained mixture was incubated at 37°C for 3 hours to effect IVT reaction and uracil or thymine specific cleavage. The obtained reaction product was purified with Clean Resin (SEQUENOM) to obtain a sample for mass spectrometry. Samples derived from genomic DNA extracted from the above clinical specimens were designated as measurement samples and samples derived from control genomic DNA were designated as control samples, both of which were used for mass spectrometry described hereinbelow.

### (2-3) Analysis of methylation status by mass spectrometry using MassARRAY^{®}

### (i) Preparation of calibration curve

Two mass spectrometric analyses were carried out independently on the control samples obtained as above. On the basis of the obtained analysis results calibration curves were prepared for the respective primer sets and correlation coefficients were calculated. Accordingly it was verified that the primer sets used could amplify both methylated DNA and non-methylated DNA without bias.

### (ii) Analysis of measurement samples

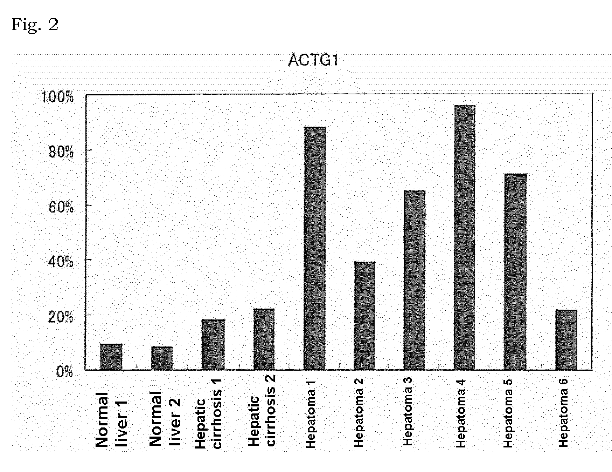

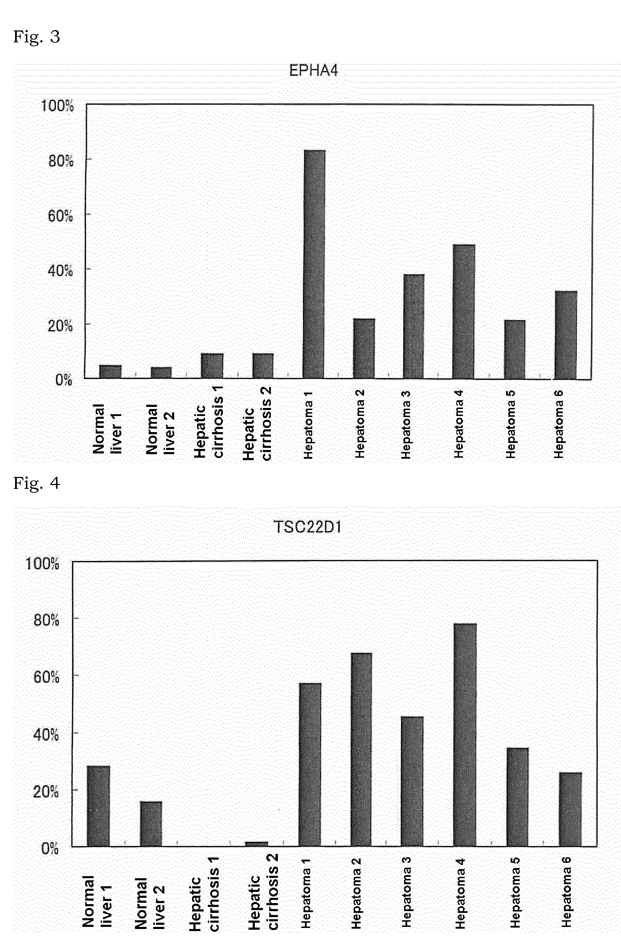

The measurement samples obtained as above were subjected to mass spectrometry to obtain peaks of DNA fragments contained in the measurement samples. The obtained peaks were then allocated to the portions of the base sequences of the markers identified in Example 1. The methylation rate was calculated on the basis of the ratio between the area of the peaks of the fragments containing a methylated CpG site and the area of the peaks of the fragments without the methylated CpG site, both fragments having the same base sequence. The obtained results are shown in Figs. 2 to 4. In these figures the vertical axis indicates the methylation ratio (%). Normal liver 1 and 2 represent normal liver tissues (2 specimens), Hepatic cirrhosis 1 and 2 represent hepatic cirrhosis tissues (2 specimens) and Hepatoma 1 to 6 represents cancerous tissues of hepatocellular carcinoma (6 specimens).

Figs. 2 to 4 reveal that the methylation rate of the present markers is significantly higher in the cancerous tissues of hepatocellular carcinoma than in normal liver tissues or hepatic cirrhosis tissues. Thus it is found that the markers are specifically methylated in cancerous tissues of hepatocellular carcinoma.

Accordingly analysis of methylation status of the present markers allows detection of cancer cells derived from hepatocellular carcinoma by distinguishing between the cancer cells and normal liver tissues or non-cancerous hepatocytes such as cells derived from liver with hepatic cirrhosis.

Consequently it is demonstrated that analysis of methylation status of a CpG site in the present markers in DNA extracted from a biological sample allows determination of the presence or absence of a cancer cell derived from hepatocellular carcinoma in the biological sample.

### Comparative Example: Comparison of performances of the present markers and known markers

Methylation status of DUSP4, ZNF141, SFRP1 and GSTP1 genes (hereinafter also referred to as "known markers") which have been known to be highly methylated in cancer cells derived from hepatocellular carcinoma was analyzed in the similar manner as Example 2.

The biological samples used were the same clinical specimens as in Example 2. Measurement samples for known markers were prepared in the same manner as Example 2 except that the following primer sets were used. The sequences of the primer sets for known markers are shown in Table 3.

**Table 3**

| **Marker** | **Forward primer** | **SEQ ID NO:** | **Reverse primer** | **SEQ ID NO:** |
|---|---|---|---|---|
| DUSP4 | TTTATTTTTGAGTAGGTAGATGT | 9 | AAACCTAAAACAAATCCTACC | 10 |
| 2NF141 | TTATAGAGTTTAGTTGGGTGTATAGT | 11 | AACACACTCTCAACCCACCT | 12 |
| SFRP1 | GTTTTGTTTTTTAAGGGGTGTTGAG | 13 | ACACTAACTCCRAAAACTACAAAAC | 14 |
| GSTP1 | GTTTTATGTTGGGAGTTTTGA | 15 | ATCCTCTTCCTACTATCTATTTACTC | 16 |

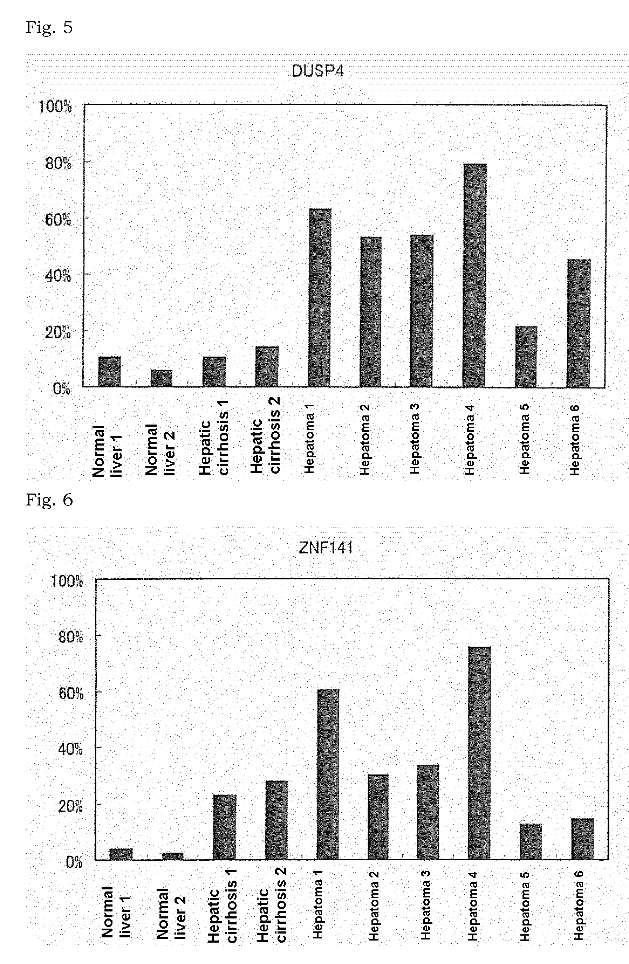

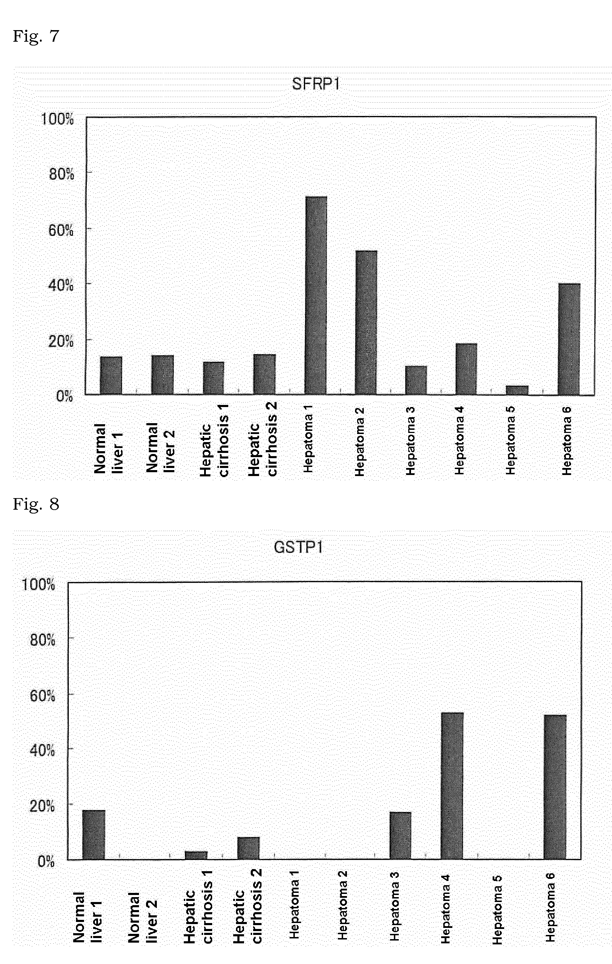

The obtained measurement samples were subjected to mass spectrometry using MassARRAY^{®} in the same manner as Example 2. The obtained results are shown in Figs. 5 to 8.

Figs. 5 to 8 reveal that the methylation rate of the known markers is significantly higher in the cancerous tissues of hepatocellular carcinoma than in normal liver tissues and hepatic cirrhosis tissues. Comparison of the result of the known markers with the result of the present markers shown in Example 2 indicates that the present markers can detect cancer cells derived from hepatocellular carcinoma with the accuracy that is the same or higher than that of the known markers.

### Example 3: Assessment of performance of present markers by MSP method

### (3-1) Biological samples

In the present Example, the biological samples used were the same normal liver tissues (2 specimens), hepatic cirrhosis tissues (5 specimens) and cancerous tissues of hepatocellular carcinoma (6 specimens) as Example 1.

### (3-2) Preparation of measurement samples and control samples (i) Extraction of genomic DNA

Genomic DNA was extracted from the above tissues with a QIAamp DNA Mini Kit (QIAGEN). The genomic DNA contained in the obtained DNA solution was fragmented with a Bioruptor (COSMO BIO).

The control genomic DNA used was genomic DNA from human peripheral blood lymphocytes. The genomic DNA from human peripheral blood lymphocytes was amplified with a GenomiPhi v2DNA amplification kit (GE Healthcare Life Sciences). The obtained amplification product consisted of non-methylated DNA. The amplification product was fragmented with a Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA).

### (ii) Bisulfite treatment

The respective DNA fragments (500 ng) obtained as above were subjected to bisulfite treatment with an EZ DNA Methylation Kit (Zymo Research) and the treated genomic DNA was dissolved in sterilized distilled water (80 µl).

### (3-3) Methylation specific PCR (MSP)

MSP was carried out on the measurement samples and control samples (DNA after bisulfite treatment) obtained in the above section (3-2). The composition of the PCR reagents, primer sets and reaction conditions for PCR in MSP are shown below.

### <PCR reagent>

| | |
|---|---|
| DDW (sterilized water) | 16.8 µL |
| 10x PCR buffer with MgCl₂ (Roche) | 2.5 µL |
| 2 mM dNTP mix | 2.5 µL |
| 10 µM sense primer | 1.0 µL |
| 10 µM anti-sense primer | 1.0 µL |
| Faststart Taq polymerase (Roche) | 0.2 µL |
| Measurement sample or control sample | 1.0 uL |
| Total | 25 µL |

### <Primer set>

The primer sets used for MSP are shown in Table 4. These primer sets allow generation of amplification products when DNA in the target regions is methylated. A primer set for accuracy control was also used which allows determination on whether or not the bisulfite treatment has been appropriately carried out (see Table 5). The base sequences of the regions which are analyzed with the primer sets shown in Table 4 in the promoter regions of ACTG1, EPHA4 and TSC22D1 genes are respectively shown in SEQ ID NOs: 31 to 33.

**Table 4**

| **Gene symbol** | **Primer** | **Base sequence of primer** | **SEQ ID NO:** | **Size of PCR product (bp)** | **Annealing temp. (°C)** | **Cycles** |
|---|---|---|---|---|---|---|
| ACTG1 | ACTG1_MF | GTAGAGCGTTTAGATTGATTC | 20 | 79 | 56 | 34 |
| | ACTG1_MR | CTAACGTTACAAAACCCCG | 21 | | | |
| EPHA4 | EPHA4_MF | CGTCGTAAATTTCGAAGAGATAC | 22 | 94 | 52 | 36 |
| | EPHA4_MR | AAATAAAAACGACAAAAACAACGTT | 23 | | | |
| TSC22D1 | TSC22D1_MF | TTTGTTGTATTGAGAGTAGAGGTGT | 24 | 92 | 57 | 36 |
| | TSC2D1_MR | AACATTAACACTAAAAAAATAACACA | 25 | | | |

**Table 5**

| **Base sequence of primer** | | **SEQ ID NO:** | **Size of PCR product (bp)** | **Annealing temp. (°C)** | **Cycles** |
|---|---|---|---|---|---|
| **Forward** | GGGATATTAAGTGGAGTTATTTTGGTTTTAGTT | 26 | 129 | 60 | 40 |
| **Reverse** | CCCTCCCAACATCCTTCCTAA | 27 | | | |

### <PCR reaction conditions>

95°C for 6 minutes;
Y cycles of 95°C for 30 seconds, X°C for 30 seconds and 72°C for 30 seconds;
72°C for 7 minutes; and
keep at 16°C.

In the above reaction conditions, "X" and "Y" respectively represent the annealing temperature and the number of cycles as indicated in Tables 4 and 5.

### (3-4) Analysis of results of methylation specific PCR (MSP)

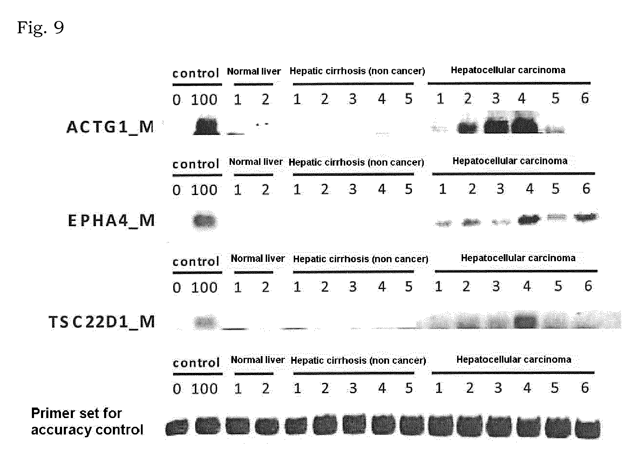

The amplification product from MSP was verified by 2% agarose gel electrophoresis. The results are shown in FIG. 9. In this figure, "0" and "100" under "control" represent the 0% methylation control sample and the 100% methylation control sample, respectively.

Fig. 9 shows that the samples from PCR using the primer set for accuracy control showed bands in all lanes. This shows that bisulfite treatment of the samples was appropriately carried out. In PCR for the present markers, bands derived from methylated CpGs were not detected for any normal liver tissues or hepatic cirrhosis tissues. In contrast, bands were detected in 5 samples, 4 samples and 3 samples in 6 samples of hepatocellular carcinoma tissues in PCR for ACTG1, EPHA4 and TSC22D1, respectively. Accordingly it is demonstrated that in methylation analysis of the present markers by MSP method, methylation of the present markers and hepatocellular carcinoma are correlated as the result of the Infinium method from Example 1. Namely it is suggested that ACTG1, EPHA4 and TSC22D1 are the markers which tend to be highly methylated in hepatocellular carcinoma.

### Example 4: MSP analysis using plasma samples

### (4-1) Biological samples

The biological samples used in the present Example were plasma (6 specimens) separated from peripheral blood collected from hepatocellular carcinoma patients. The control samples used were plasma (3 specimens) separated from peripheral blood from healthy subjects.

### (4-2) Preparation of measurement samples and control samples (i) Extraction of genomic DNA

Genomic DNA was extracted from the plasma (2 mL) with a QIAamp Circulating Nucleic Acid Kit (QIAGEN).

### (ii) Bisulfite treatment

The genomic DNA obtained as above was subjected to bisulfite treatment with an EZ DNA Methylation Kit (Zymo Research) and the treated genomic DNA was dissolved in sterilized distilled water (80 µl).

### (4-3) MSP

MSP was carried out on the measurement samples obtained in the above section (4-2). The composition of the PCR reagent, primer sets and reaction conditions for PCR in MSP are shown below.

### <PCR reagent>

| | |
|---|---|
| DW (sterilized water) | 16.8 µL |
| 10 x PCR buffer with MgCl₂ (Roche) | 2.5 µL |
| 2 mM dNTP mix | 2.5 µL |
| 10 µM sense primer | 1.0 µL |
| 10 µt anti-sense primer | 1.0 µL |
| Faststart Taq polymerase (Roche) | 0.2 µL |
| Measurement sample | 1.0 uL |
| Total | 25 µL |

### <Primer set>

The primer sets used for MSP are shown in Table 6.

**Table 6**

| **Primer** | | **Base sequence of primer** | **SEQ ID NO:** | **Annealing temp. (°C)** | **Cycles** |
|---|---|---|---|---|---|
| TSC22D1 | Forward | TATTTGTTGTATTGAGAGTAGAGGC | 34 | 59 | 50 |
| | Reverse | AAACATTAACGCTAAAAAAATAACG | 35 | | |
| EPHA4 | Forward | CGTCGTAAATTTCGAAGAGATAC | 22 | 52 | 50 |
| | Reverse | AAATAAAAACGACAAAAACAACGTT | 23 | | |
| Accuracy control | Forward | GGGATATTAAGTGGAGTTATTTTGGTTTTAGTT | 26 | 60 | 40 |
| | Reverse | CCCTCCCAACATCCTTCCTAA | 27 | | |

### <PCR reaction conditions>

95°C for 6 minutes;
Y cycles of 95°C for 30 seconds, X°C for 30 seconds and 72°C for 30 seconds;
72°C for 7 minutes; and
keep at 16°C.

In the above reaction conditions, "X" and "Y" respectively represent the annealing temperature and the number of cycles as indicated in Table 6.

### (4-4) Analysis of results of MSP

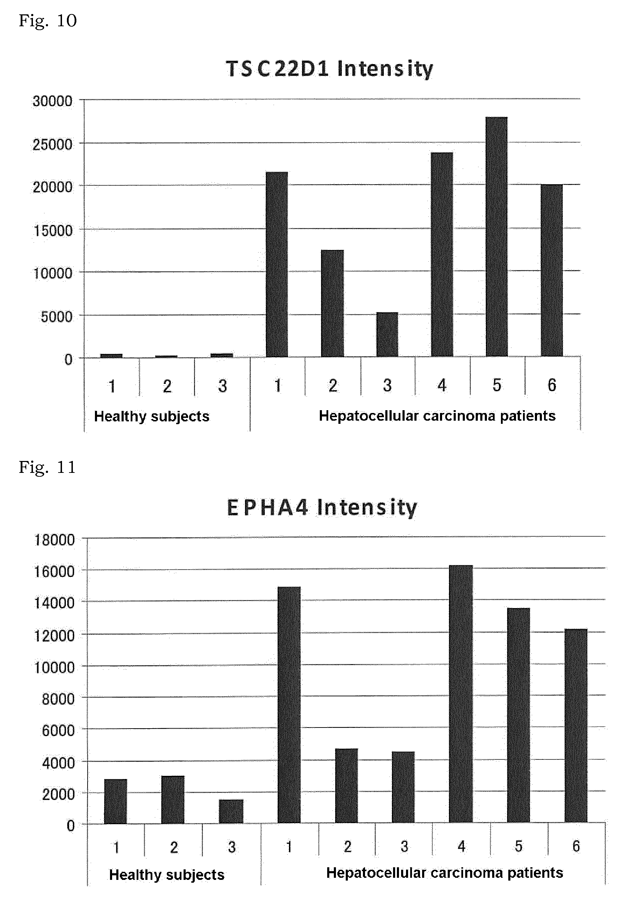

The amplification product from MSP was verified by 2% agarose gel electrophoresis. The intensity of the bands visualized by agarose gel electrophoresis was quantified with a tool from ImageJ (open source; available from http://rsbweb.nih.gov/ij/). The value of the background was subtracted from the value of the band of a sample in each electrophoresis lane to calculate the detection value which was used to generate a graph. The results are shown in Figs. 10 to 12.

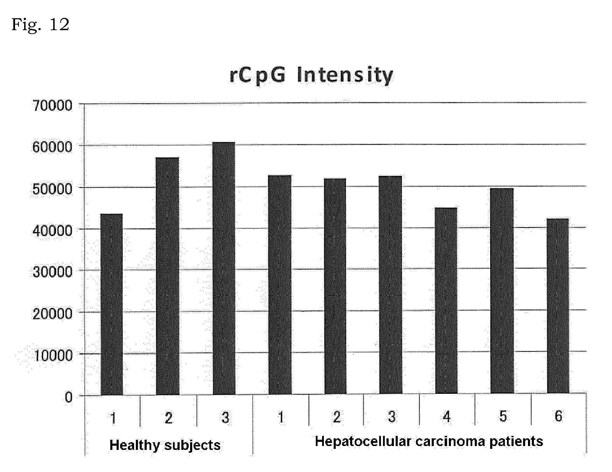

Fig. 10 indicates that in PCR for TSC22D1 bands were not detected for plasma from healthy subjects while bands were detected in all 6 specimens of plasma from hepatocellular carcinoma patients. Fig. 11 indicates that in PCR for EPHA4 bands were not detected for plasma from healthy subjects while bands were detected in 4 specimens among 6 specimens of plasma from hepatocellular carcinoma patients. Fig. 12 shows that in PCR using the primer set for accuracy control, bands were detected for all samples. This indicates that bisulfite treatment of the samples was appropriately carried out. Accordingly the present markers tend to be methylated in hepatocellular carcinoma patients and are not detected to be methylated in healthy subjects even when the biological sample used is plasma. Thus it is suggested that the present markers have high specificity towards hepatocellular carcinoma.

## Claims

1. A method for determining the presence or absence of a cancer cell derived from hepatocellular carcinoma comprising the steps of:
extracting DNA from a biological sample collected from a subject;
analyzing methylation status of a CpG site in a promoter region of at least one gene selected from ACTG1, EPHA4 and TSC22D1 in the DNA obtained from the extraction step; and
determining the presence or absence of a cancer cell derived from hepatocellular carcinoma in the biological sample based on an analysis result obtained from the analyzing step.

2. The method according to claim 1, wherein the analyzing step is the step of analyzing whether or not at least one CpG site is methylated.

3. The method according to claim 2, wherein the determination step is the step of determining that the biological sample contains the cancer cell when the analysis result shows that a CpG site is methylated.

4. The method according to claim 1, wherein the analyzing step is the step of analyzing a methylation frequency.

5. The method according to claim 4, wherein the determination step is the step of determining that the biological sample contains the cancer cell when the methylation frequency obtained from the analyzing step is higher than a predetermined threshold.

6. Use of a marker for determining the presence or absence of a cancer cell derived from hepatocellular carcinoma by methylation analysis, which is at least one CpG site selected from CpG sites located in promoter regions of ACTG1, EPHA4 and TSC22D1 genes.

7. Use of a kit for determining the presence or absence of a cancer cell derived from hepatocellular carcinoma, said kit comprising a primer set for analysis of methylation status of at least one CpG site selected from CpG sites located in promoter regions of ACTG1, EPHA4 and TSC22D1 genes.

8. The use according to claim 7, wherein the primer set is a primer set for analyzing methylation status of the CpG site by at least one method selected from mass spectrometry and methylation specific PCR method.

9. The use according to claim 8, wherein the primer set is at least one selected from:
a primer set of primers respectively having base sequences SEQ ID NO: 1 and SEQ ID NO: 2;
a primer set of primers respectively having base sequences SEQ ID NO: 3 and SEQ ID NO: 4;
a primer set of primers respectively having base sequences SEQ ID NO: 5 and SEQ ID NO: 6;
a primer set of primers respectively having base sequences SEQ ID NO: 20 and SEQ ID NO: 21;
a primer set of primers respectively having base sequences SEQ ID NO: 22 and SEQ ID NO: 23;
a primer set of primers respectively having base sequences SEQ ID NO: 24 and SEQ ID NO: 25; and
a primer set of primers respectively having base sequences SEQ ID NO: 34 and SEQ ID NO: 35.

## Patentansprüche

1. Verfahren zur Bestimmung des Vorliegens oder Fehlens einer aus hepatozellulärem Karzinom stammenden Krebszelle, umfassend die Schritte:
Extrahieren von DNA aus einer von einem Individuum erhaltenen biologischen Probe;
Analysieren des Methylierungsstatus einer CpG-Stelle in einer Promotorregion wenigstens eines aus ACTG1, EPHA4 und TSC22D1 ausgewählten Gens in der aus dem Extraktionsschritt gewonnenen DNA; und
Bestimmen des Vorliegens oder Fehlens einer aus hepatozellulärem Karzinom stammenden Krebszelle in der biologischen Probe anhand eines aus dem Analyseschritt gewonnenen Analyseergebnisses.

2. Verfahren nach Anspruch 1, wobei es sich bei dem Analyseschritt um den Schritt handelt, bei dem analysiert wird, ob wenigstens eine CpG-Stelle methyliert ist oder nicht.

3. Verfahren nach Anspruch 2, wobei es sich bei dem Bestimmungsschritt um den Schritt handelt, bei dem bestimmt wird, dass die biologische Probe die Krebszelle enthält, wenn das Analyseergebnis zeigt, dass eine CpG-Stelle methyliert ist.

4. Verfahren nach Anspruch 1, wobei es sich bei dem Analyseschritt um den Schritt handelt, bei dem eine Methylierungshäufigkeit analysiert wird.

5. Verfahren nach Anspruch 4, wobei es sich bei dem Bestimmungsschritt um den Schritt handelt, bei dem bestimmt wird, dass die biologische Probe die Krebszelle enthält, wenn die aus dem Analyseschritt gewonnene Methylierungshäufigkeit höher als ein vorbestimmter Schwellenwert ist.

6. Verwendung eines Markers zur Bestimmung des Vorliegens oder Fehlens einer aus hepatozellulärem Karzinom stammenden Krebszelle durch Methylierungsanalyse, wobei es sich um wenigstens eine aus CpG-Stellen, die in Promotorregionen von ACTG1-, EPHA4- und TSC22D1-Genen lokalisiert sind, ausgewählte CpG-Stelle handelt.

7. Verwendung eines Kits zur Bestimmung des Vorliegens oder Fehlens einer aus hepatozellulärem Karzinom stammenden Krebszelle, wobei das Kit einen Primersatz zur Analyse wenigstens einer aus CpG-Stellen, die in Promotorregionen von ACTG1-, EPHA4- und TSC22D1-Genen lokalisiert sind, ausgewählten CpG-Stelle umfasst.

8. Verwendung nach Anspruch 7, wobei es sich bei dem Primersatz um einen Primersatz zur Analyse des Methylierungsstatus der CpG-Stelle mit wenigstens einem aus Massenspektrometrie und methylierungsspezifischem PCR-Verfahren ausgewählten Verfahren handelt.

9. Verwendung nach Anspruch 8, wobei es sich bei dem Primersatz um wenigstens einen aus den Folgenden ausgewählten handelt:
ein Primersatz von Primern mit den Basensequenzen SEQ ID NO: 1 bzw. SEQ ID NO: 2;
ein Primersatz von Primern mit den Basensequenzen SEQ ID NO: 3 bzw. SEQ ID NO: 4;
ein Primersatz von Primern mit den Basensequenzen SEQ ID NO: 5 bzw. SEQ ID NO: 6;
ein Primersatz von Primern mit den Basensequenzen SEQ ID NO: 20 bzw. SEQ ID NO: 21;
ein Primersatz von Primern mit den Basensequenzen SEQ ID NO: 22 bzw. SEQ ID NO: 23;
ein Primersatz von Primern mit den Basensequenzen SEQ ID NO: 24 bzw. SEQ ID NO: 25; und
ein Primersatz von Primern mit den Basensequenzen SEQ ID NO: 34 bzw. SEQ ID NO: 35.

## Revendications

1. Procédé de détermination de la présence ou de l'absence d'une cellule cancéreuse issue d'un carcinome hépatocellulaire comprenant les étapes suivantes :
extraction de l'ADN d'un échantillon biologique prélevé chez un sujet ;
analyse de l'état de méthylation d'un site CpG dans une région promotrice d'au moins un gène choisi parmi ACTG1, EPHA4 et TSC22D1 dans l'ADN obtenu suite à l'étape d'extraction ; et
détermination de la présence ou de l'absence d'une cellule cancéreuse issue d'un carcinome hépatocellulaire dans l'échantillon biologique sur la base du résultat de l'analyse obtenu suite à l'étape d'analyse.

2. Procédé selon la revendication 1, dans lequel l'étape d'analyse consiste en une étape d'analyse visant à déterminer si au moins un site CpG est méthylé ou pas.

3. Procédé selon la revendication 2, dans lequel l'étape de détermination consiste en une étape visant à déterminer que l'échantillon biologique contient bien la cellule cancéreuse lorsque le résultat de l'analyse montre qu'un site CpG est méthylé.

4. Procédé selon la revendication 1, dans lequel l'étape d'analyse consiste en une étape d'analyse de la fréquence de méthylation.

5. Procédé selon la revendication 4, dans lequel l'étape de détermination consiste en une étape visant à déterminer que l'échantillon biologique contient bien la cellule cancéreuse quand la fréquence de méthylation obtenue à l'issue de l'étape d'analyse est supérieure à un seuil prédéterminé.

6. Utilisation d'un marqueur pour déterminer la présence ou l'absence d'une cellule cancéreuse issue d'un carcinome hépatocellulaire par analyse de la méthylation, ledit marqueur étant au moins un site CpG choisi parmi les sites CpG situés dans les régions promotrices des gènes ACTG1, EPHA4 et TSC22D1.

7. Utilisation d'un kit pour déterminer la présence ou l'absence d'une cellule cancéreuse issue d'un carcinome hépatocellulaire, ledit kit comprenant un ensemble d'amorces pour l'analyse de l'état de méthylation d'au moins un site CpG choisi parmi les sites CpG situés dans les régions promotrices des gènes ACTG1, EPHA4 et TSC22D1.

8. Utilisation selon la revendication 7, dans laquelle l'ensemble d'amorces est un ensemble d'amorces pour l'analyse de l'état de méthylation du site CpG par au moins un procédé choisi parmi la spectrométrie de masse et le procédé de PCR spécifique de la méthylation.

9. Utilisation selon la revendication 8, dans laquelle l'ensemble d'amorces est au moins un ensemble d'amorces choisi parmi :
un ensemble d'amorces comportant respectivement les séquences de bases SEQ ID NO : 1 et SEQ ID NO : 2 ;
un ensemble d'amorces comportant respectivement les séquences de bases SEQ ID NO : 3 et SEQ ID NO : 4 ;
un ensemble d'amorces comportant respectivement les séquences de bases SEQ ID NO : 5 et SEQ ID NO : 6 ;
un ensemble d'amorces comportant respectivement les séquences de bases SEQ ID NO : 20 et SEQ ID NO : 21 ;
un ensemble d'amorces comportant respectivement les séquences de bases SEQ ID NO : 22 et SEQ ID NO : 23 ;
un ensemble d'amorces comportant respectivement les séquences de bases SEQ ID NO : 24 et SEQ ID NO : 25 ;
un ensemble d'amorces comportant respectivement les séquences de bases SEQ ID NO : 34 et SEQ ID NO : 35.
